# EUROPEAN PATENT SPECIFICATION

(11) **EP 3 003 251 B1**
(45) Date of publication and mention of the grant of the patent: **10.07.2019**
(21) Application number: 13742503.9
(22) Date of filing: 06.06.2013
(51) Int. Cl.: A61K 8/25, A61K 8/29, A61K 8/49, A61K 8/58, A61Q 11/00, A61K 9/16, A61K 33/38, A61L 15/46, A61K 8/43, A61Q 17/00, A61K 9/00, A61K 9/14, A61K 8/02, A61K 8/19, A61K 31/28, A61L 26/00, A61K 31/555, A61K 33/00, C07F 7/02, A61P 31/00

(54) **ANTIBACTERIAL POWDERS BASED ON ANIONIC SILICON OR TITANIUM DIOXIDE ADSORBED WITH PHARMACEUTICALLY ACTIVE CATIONS**
ANTIBAKTERIELLE PULVER AUF BASIS VON SILICIUM ODER TITANDIOXID, AN DIE PHARMAZEUTISCH AKTIVE KATIONEN ADSORBIERT SIND
POUDRES ANTIBACTÉRIENNES BASÉES SUR DU DIOXYDE DE SILICIUM OU TITANE ANIONIQUE ADSORBÉ AVEC DES CATIONS PHARMACEUTIQUEMENT ACTIFS

(43) Date of publication of application: 13.04.2016
(73) Proprietor: Pavia Farmaceutici S.r.l., 27010 Copiano (Pavia) (IT)
(72) Inventor: FERRARI, Massimo, I-27010 Copiano (pavia) (IT)
(74) Representative: Biggi, Cristina
(86) International application number: PCT/IB2013/054647
(87) International publication number: WO 2014/195762

(56) References cited:
- EP-A- 0 251 783
- EP-A2- 1 825 752
- WO-A1-2007/079841
- WO-A1-2008/043396
- VERRAEDT E ET AL: "Controlled release of chlorhexidine from amorphous microporous silica", JOURNAL OF CONTROLLED RELEASE, ELSEVIER, AMSTERDAM, NL, vol. 142, no. 1, 25 February 2010 (2010-02-25), pages 47-52, XP026892473, ISSN: 0168-3659, DOI: 10.1016/J.JCONREL.2009.09.022 [retrieved on 2009-10-03]
- BARBOUR ET AL: "Chlorhexidine adsorption to anatase and rutile titanium dioxide", COLLOIDS AND SURFACES. A, PHYSICACHEMICAL AND ENGINEERING ASPECTS, ELSEVIER, AMSTERDAM, NL, vol. 307, no. 1-3, 7 August 2007 (2007-08-07), pages 116-120, XP022188492, ISSN: 0927-7757, DOI: 10.1016/J.COLSURFA.2007.05.010
- KAWASHITA M ET AL: "Preparation of silver-doped silica glass microspheres", JOURNAL OF BIOMEDICAL MATERIALS RESEARCH. PART A, WILEY PERIODICALS INC, HOBOKEN, NY, US, vol. 66A, no. 2, August 2003 (2003-08), pages 266-274, XP002467425, ISSN: 1549-3296, DOI: 10.1002/JBM.A.10547

## Description

The present invention generally refers to anionic silicon or titanium dioxide particles, adsorbed with one or more pharmaceutical active cations, particularly useful as antibacterial, antiviral or antimycotic agent.

### Background

A number of chemical techniques have been applied to the study of the acid-base equilibria at the surface of metal or metalloidic oxides, abbreviated as M-0. The chemistry of an oxide surface in contact with an aqueous solution is determined to large extent to the protonation or dissociation of the surface hydroxyl groups, as schematically shown by the following equations:

M-OH₂⁺ ↔ M-OH ↔ MO⁻ + H⁺

← decreasing pH increasing pH →

The two equilibria indicate that by appropriate adjustment of pH, the surface of the metal oxide may carry either a positive or a negative charge, while at an intermediate value the hydroxyl groups are undissociated and the surface has zero charge. The point of zero charge (pzc) defines the situation when there is net zero charge on the surface, determinable, e.g., by potentiometric titrations. The pH at the pzc has a specific value for each metal oxide depending on the electrostatic character of the metal-oxigen bond. In the case of silica (SiO₂), the point of zero charge have been reported in the range of pH 1-2 (G.D. Parfitt, Pure & Appl. Chem. Vol 48, 415-418, 1976; G. A. Parks, Chem. Rev, Vol 65, 177, 1965)
In addition, the dissociation constant (pKa) of the silanol groups (Si-OH), which are present at the silica surface, has been determined in the range 4-5.5 (L. H. Allen et al. J. Inorg. Nucl. Chem. Vol 33, 1293, 1971; S.W. Ong et al. Chem. Phys. Lett. Vol 192, 327, 1992) indicating that dissociation of Si-OH groups to form negatively charged SiO⁻ groups at the surface of silica may occur.

Aqueous suspensions of negatively charged silica particles, (colloidal silica) are known in the art and the maximum weight fraction of silica is typically limited on the base of the average particle size, which ranges from about 3 nm to about 150 nm. Dispersions with small silica particles (<10 nm) are normally quite clear. Midsize dispersion (10-20 nm) start to take on an opalescent appearance as more light is scattered. Dispersion containing large colloidal silica particles (>50 nm) are normally white.

Colloidal dispersions are stable against gelling and settling in the pH range 8-10.5. These colloidal silicas are charge stabilized by cations, normally of sodium, potassium, or ammonia and are commercially available, for example, from Companies such as AkzoNobel, Nissan Chemical, and Evonik. Colloidal silica suspensions have a number of interesting applications which may include for example, papermaking, abrasive, concrete densifier, catalysts, refractory, ceramic fiber stabilizer, antisoiling, etc.

Controlled release of chlorhexidine antiseptic from microporous amorphous silica has ben recenty proposed by E. Verraedt et al (J. Control Release 142(1):6, 2010) and applied in open porosity of an implant surface. The amorphous microporous silica (AMS) was serving as a reservoir for controlled release of a bioactive agent in the open porosity of a titanium coating. The pores of the AMS emptied by calcination were loaded with chlorhexidine diacetate (CHX) via incipient wetness impregnation with CHX solution, followed by solvent evaporation. Using this CHX loaded AMS system on titanium substrate sustained release of CHX into physiological medium was obtained over a 10 day-period. CHX released from the AMS coating was demonstrated to be effective in killing planktonic cultures of the human pathogens Candida albicans and Staphylococcus epidermidis. It was suggested that this surface modification of titanium bodies with AMS controlled release functionality for a bioactive compound potentially can be applied on dental and orthopaedic implants to abate implant-associated microbial infection.

However, this approach is considerably different from that described in this invention since it makes use of a silica, produced by acidic hydrolysis, which cannot bind as a consequence chlorhexidine through an electrostatic interaction, differently from what presently described and claimed.

Document WO2008043396A1 describes a composition wherein Ag⁺ or Cu⁺ are adsorbed through a ligand with a negative charge onto particles of titanium dioxide or colloidal silica. Barbour et. al. (Colloids and Surfaces A: Physicochem. Eng. Aspects 307 (2007) 116-120) reports a study on the adsorption and subsequent desorption of a common antimicrobial, chlorhexidine digluconate (CHX), to anatase and rutile titanium dioxide investigated in two different buffers. At the pH of the chlorhexidine solution used in these investigations (pH 6.02), a small negative charge on anatase and a larger negative charge on rutile would be expected while CHX, which is a weak base, carries a positive charge at this pH.

Kawashita et. al. (Journal of Biomedical Materials Research Part A, Wiley Periodicals Inc. 66A(2) (2003) 266-274) reports the preparation of silver doped silica glass microspheres having a diameter of 1pm, using the sol-gel method. After the preparation of the silica substrate, an ammonia solution containing silver nitrate is added to form silica microspheres doped with silver ion together with aluminum ions (Si/Al/Ag = 1/0.01/0.01) which show antibacterial activity against Escherichia coli.

Document EP0251783A is concerned with an antimicrobial composition that comprises an antimicrobial ionic silver compound deposited on a physiologically inert oxidic synthetic particulate support material as TiO₂.

In document WO2007/079841A1 nanoscalar particles (1-2000 nm) based on SiO₂ presenting a cationic antimicrobial on their surface as a quaternary ammonium salt are described. Document EP1825752A2 reports the combination of a Si02 - generating agent together with special antimicrobial agents selected from two out of three different classes of substances. Said antibacterial agent being selected from at least two compounds of the three following compound classes in the form of cationic, anionic or nonionic: deacetylated chitosans and chitosan derivatives and/or phenols of the group of halogenated dihydroxydiphenylmethanes, sulfides, and ethers and/or substituted quarterly ammonium salts of phosphoric acid alkylation.

The need thus arises to provide a new product based on anionic dioxide particles, that is efficacious, e.g., in treating infections and inflammations, has a good cutaneous tolerance, and may form an efficacious barrier against external microbes, so avoiding any infection on the affected epithelium.

### Summary of the invention

In a first aspect, the invention refers to a derivative of formula (I):

**(MO₂)**⁻ (**A**^{m+})_{y} **(I)**

wherein:
- **M**: is Si or Ti;
- **(MO₂)⁻**: represents an anionic dioxide particle comprising tetrahedral Silicon or octahedral Titanium atoms, interconnected by bridging oxygen atoms,
- **A**^{m+}: represents a chlorhexidine cation and a cationic silver complex of formula Ag-L, having L preferably selected from: optionally substituted 8-mercaptoquinoline hydrochloride and 2-mercapto-4-(C1-C6Alk)pyrimidine hydrochloride , A^{m+} being linked to the anionic silicon or titanium dioxide particle by electrostatic interaction, wherein **m** is the charge of the active species and it is comprised from 1 to 20, preferably from 1 to 10,
- **Y**: represents the number of different active species A^{m+} co-adsorbed on the same anionic silicon or titanium dioxide particle and it is equal to 2.

In a preferred embodiment, M is Si, and the (MO₂)⁻ represents anionic silica particles (SiO₂)⁻.

In another preferred embodiment, M is Ti and (MO₂)⁻ represents titanium dioxide anionic particles (TiO₂)⁻.

The present derivatives are characterised by the fact that the anionic negatively charged particles (MO₂)⁻, preferably anionic silica particles, have an average particle size (ps), intended as average diameter, of at least 200 nm, and that the positive A^{m+} species is adsorbed on the surface of said anionic particles by electrostatic interactions.

In a further aspect, the invention also refers to a process for the preparation of the above identified derivatives of formula (I), comprising the reaction of anionic particles of general formula (MO₂)⁻ having a ps of at least 200 nm, with two positively charged A^{m+} species, in the presence of an alcoholic solvent system.

In another aspect, the invention refers to a derivative of general formula (I), preferably where M=Si, for use as a medicament, in particular as antibacterial, antimycotic, antiviral and/or sporicidal agent.

In another aspect, the present invention also relates to a composition comprising the above indicated derivatives of formula (I), in admixture with at least one physiologically acceptable excipient or carrier, and optionally, with another active ingredient. In particular, said composition can be in the form of a cream, a gel or a powder. This composition is suitable for the topical administration route, mainly intended for the use as antibacterial, antimycotic and antiviral agent.

### Description of the drawings

Figure 1: schematic representation of the anionic silica particle (SiO₂)⁻.
Figure 2: average particle size (ps) distribution of a derivative of formula (I) wherein: M=Si and A^{m+}= NH₄⁺ cation, according to Example 1.
Figure 3: Zeta potential of a derivative of formula (I), wherein M=Si and A^{m+}= NH₄⁺ cation, according to Example 1.
Figure 4: average particle size (ps) distribution of a derivative of formula (I) of the invention, wherein M=Si and A^{m+} is the chlorhexidine cation, according to Example 2.
Figure 5: Zeta potential of a derivative of formula (I) of the invention, wherein M=Si and A^{m+} chlorhexidine cation, according to Example 2.

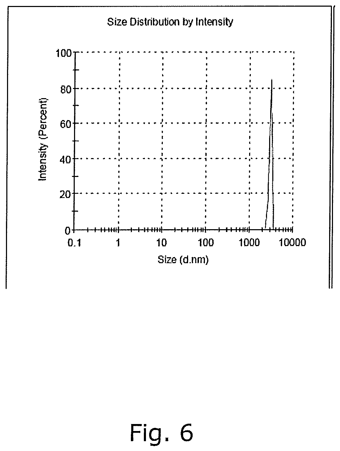

Figure 6: average size distribution of 3000 ± 360 nm of Si02 particles with the electrostatically bound polymeric PHMB, according to Example 5.
Figure 7: Zeta potential of the derivatives of Example 5.
Figure 8: Petri capsule showing the antimicrobic activity of comparable amounts of powders composed by anionic derivatives of formula (I), where M=Si and A^{m+} is: the ammonium cation (A); the silver complex [Ag-L]⁺ of example 3 (B);
   the chlorhexidine cation, according to example 2 (C) and a mixture of co-adsorbed silver complex Ag-L⁺ and chlorhexidine cation, according to example 4 (D).

### Detailed description of the invention

According to a first aspect of the invention, there is provided a dioxide silicon or titanium derivative having the general formula (I):

**(MO₂)**⁻ **(A^{m+})y** (I)

wherein:
M Silicon or titanium, and A^{m+} represents chlorhexidine cation and a cationic silver complex of formula Ag-L, having L preferably selected from: optionally substituted 8-mercapto-quinoline hydrochloride and 2-mercapto-4-(C1-C6Alk)pyrimidine hydrochloride, A^{m+} being linked to the selected anionic, negatively charged, dioxide particle by electrostatic interaction.

In this respect, the negative charge of the anionic (MO₂)⁻ particles may have a value comprised in the range 0.01-0.2, with reference to one mole of SiO₂ or TiO₂.

Y represents the number of the different kind of active species A^{m+} co-adsorbed on the same anionic silicon or titanium particle, which can be of the same type or of different chemical nature. According to formula (I), the present derivative presents two different kind of active species A^{m+}, co-adsorbed on the same anionic particle.

According to the invention, the positively charged active species A^{m+} is preferably selected from: an antibacterial, an antimycotic, an antiviral and/or sporicidal agent, whereas antibacterial agent is particularly preferred.

In this respect, A^{m+} is at least one compound selected from the group consisting of: chlorhexidine cations.

In a more preferred embodiment, A^{m+} is chlorhexidine digluconate or acetate.

The mentioned preferred Am+ species are illustrated in the Table 1 below:

**Table 1: preferred A^{m+} species of the invention.**

| | **Chemical formula** | **Chemical name** |
|---|---|---|
| **1.** | 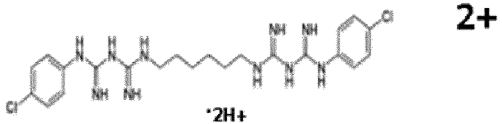 | Chlorhexidine cation |
| **2.** | 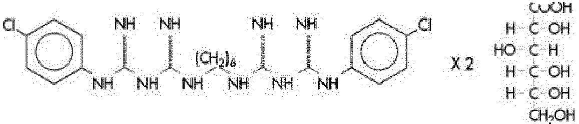 | Chlorhexidine digluconate |
| **3.** | 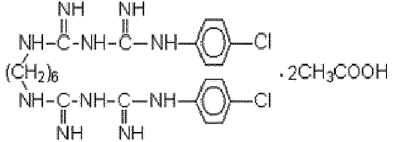 | Chlorhexidine acetate |
| **4.** | 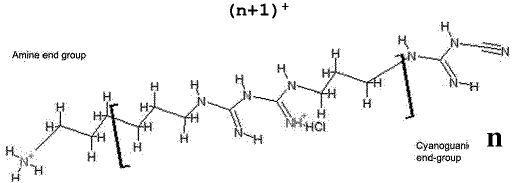 | Polyexamethylene Biguanide hydrochloride Cation(not part of the invention) |
| **5.** | 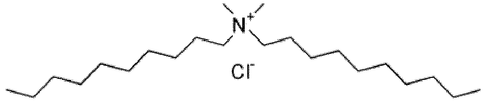 | Didecyl Dimethylammonium Cation (not part of the present invention) |

According to the invention A^{m+} is also a cationic silver complex of general formula [Ag-L]⁺, wherein L is the chelating moiety (or ligand).

L is selected from optionally substituted 8-mercapto-quinoline hydrochloride and 2-mercapto-4-(C₁-C₆-Alk)-pyrimidine, preferably 2-mercapto-4-methylpyrimidine, of formula:

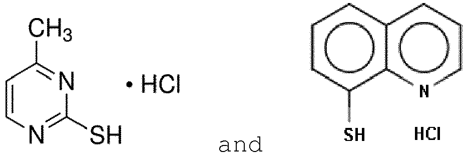

The term "C₁-C₆-Alk" means an optionally substituted linear or branched hydrocarbon chain residue, having from 1 to 6 carbon atoms, e.g.: methyl, propyl, iso-propyl, butyl, isobutyl, ter-butyl and the like.

According to the invention A^{m+} is therefore two of the above mentioned compounds, self-assembled by electrostatic forces, to the same anionic particle. In this respect, it has to be noted that when more than one kind of A^{m+} species is used (i.e. y=2), these are co-adsorbed, preferably on the same (SiO₂)⁻ particle, by electrostatic interactions, thus providing poly-functionalized anionic (silica) particles, having a broad spectrum of pharmacological activity. Preferably, the selected cationic silver complex [Ag-L]⁺ is co-adsorbed with chlorhexidine cations, preferably chlorhexidine digluconate, to form a multifunctionalised derivatives of formula (I) having y=2, and even more preferably having M=Si, as described, e.g., in the herein enclosed experimental part. In this direction, and according to a still preferred embodiment, the derivatives of formula (I) are co-adsorbed with chlorhexidine digluconate and a complex [Ag-L]⁺, wherein L is 2-mercapto-4-methylpyrimidine hydrochloride, being said derivatives particularly preferred when M=Si.

Applicant would like to note that when two active species of different chemical nature, such as the [Ag-L]+ complex and the chlorhexidine cation, are co-adsorbed on the same dioxide anionic particles they can act synergically increasing their own specific antimycrobic power. In fact, when chlorhexidine and a silver complex Ag-L are co-adsorbed, it is possible to obtain a higher antibacterial activity.

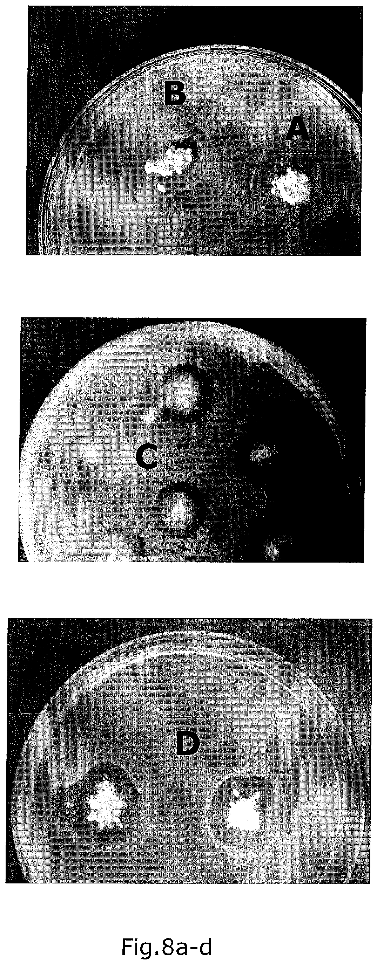

This fact is demonstrated by the results reported in Figure 8, where it can be observed that the thickness of the inhibition layer surrounding comparable amounts of powders, based on silicon derivatives of formula I, in contact with the pull of microbes described in example 6, increases more than additively upon going from A^{m+} = [Ag-L]⁺ (ca 1.2 mm), (Figure 8B), to A^{m+} = Chlorhexidine (ca. 2.6-2.8 mm), (Figure 8C), to A^{m+} = [Ag-L]⁺ + Chlorhexidine (> 5 mm), (Figure D), while no inhibition of the microbial growth is observed when A^{m+} is the ammonium cation.

In other words, this means that by co-adsorbing e.g. chlorhexidine and a silver complex on the same anionic particle, it is possible to obtain a derivative of formula (I), endowed with a higher antibacterial activity, with respect to the activity of the chlorhexidine and the silver complex when used themselves.

Advantageously, the present derivatives can contain a total amount of A^{m+} species, preferably up to 30%, more preferably comprised from 5 and 20%, with respect to the total weight of the derivative thus obtained. According to one embodiment, when the derivatives comprise a silver complex, this latter is preferably used in an amount comprised from 0.1 to 0.5%, being said percentage referred to Ag⁺ amount. By that, it is possible to obtain the present derivatives (I), e.g. in form of a powder, characterised by having multi functionalised anionic silica or titanium particles, whereby the different A^{m+} species can act synergically.

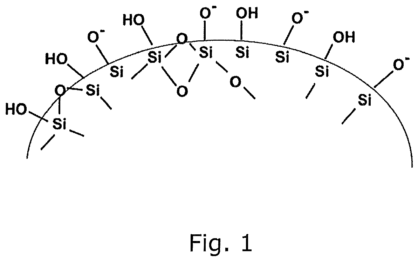

As above mentioned, the derivatives (I) of the invention have a chemical structure thereby a number of tetrahedral Si or octahedral Ti atoms are interconnected each other by bridging oxygen atoms. In this direction, Figure 1 schematically shows the tetrahedral structure of (SiO₂)⁻ particles.

In particular, it has to be noted that the present anionic silicon or titanium dioxide particles (MO₂)⁻, are characterised by the fact that they have an average particle size of at least 200nm. Preferably, the diameter of the particles (i.e. the particle size) is comprised from 200nm to 2 µm, being an average diameter comprised from 500nm and 1 µm particularly preferred. It follows that the present derivatives are not classifiable under the definition of "nano-material", since the diameter higher than 200 nm is indicative of a particle size which is not intended in the art as nano-particle. In fact, as for instance adopted by the European Commission, a nanomaterial is defined as a material having a size ranging from 1 nm to 100 nm (for a general reference about said definition, see e.g. http://ec.europa.eu/environment/chemicals/nanotech/#definitio n).

The particle size of the derivatives of the invention is particular relevant because, using particles with size higher than 200 nm allow to adsorb on the same particle an higher number of active A^{m+} species, even of different chemical nature as above reported. Even further, another advantage in using a ps higher than 200nm, is that it is possible to obtain the present derivatives in form of a powder which can be easily separated by filtration during the industrial preparation.

An additional advantage of the present antibacterial derivatives is that binding electrostatically the active species A^{m+} to the negatively charged (preferably silica) substrate, allows the preparation of antibacterial formulations, for example in the form of creams or gel, where the active specie can act as an antimicrobic barrier and is therefore very useful for the realization of Medical Devices for topical use, whereby the release of the active ingredient to the tissue should be retained or somehow controlled.

The above indicated anionic silica particles (SiO₂)⁻ may be produced, e.g., as reported in the following. In 1956, Kolbe (G. KOLBE, "*Das komplexehemische Verhalten der Kiesels-ure*" Dissertation, Jena (19564)) described the formation of silica particles by reacting tetraethyl silicate in alcoholic solution with water in the presence of bases. With very pure reactants he observed in several cases a slowly proceeding reaction leading to the formation of uniform spherical silica. Later, W. Stober et al. (Journal of Colloid and Interface Science 26, 62-69, 1968) developed a system of chemical reactions which allowed the controlled growth of spherical silica particles of uniform size by means of hydrolysis of alkyl silicates and subsequent condensation in alcoholic solutions. Ammonia was used as a morphological catalyst. Stober found that by reacting tetraethyl silicate with different amount of water ethanol and ammonium hydroxide it was possible to prepare monodispersed silica particles of different sizes.

Elemental analysis data of the silica powder obtained by hydrolysis of tetraethoxysilane in ethanol/water/ammonia reveals a content in Nitrogen higher than 0.3%, indicating the presence of ammonium cations which are needed to balance the negative charges at the surface of the solid particle. In addition the observation of a negative value of the zeta potential for silica powder suspensions in water demonstrates and gives a clear picture of the negative charge at the surface of the silica particles.

As far as the anionic titanium dioxide particles are concerned, it has to be noted that the TiO₂ exists in different allotropic forms: anastase, rutile and brookite, all of them commercially available. The oxide surface is nevertheless characterised by the presence of Ti-OH groups which may undergo deprotonation at a pH higher than 7, to form Ti-O⁻ units, after treatment with a basic substance such as ammonium hydroxide or the like.

As a general example, treatment of TiO₂ powders with an ethanol-acetone solution containing a 10-20% of ammonium hydroxide allows to obtain anionic powders which can be used as a starting substrate for the preparation of antibacterial derivatives of formula (I), e.g. in the form of a powder. Therefore, according to a further aspect, the invention refers to a process for the preparation of a derivative of formula (I), comprising the electrostatic interaction of (TiO₂)⁻ or, preferably, (SiO₂)⁻ particles having a ps of at least 200nm, with two compounds capable to generate the selected positively charged species A^{m+}, in the presence of an alcoholic solvent system. In this respect, preferred solvent systems are aqueous mixture of lower C₁-C₄ aliphatic alcohol, being ethanol particularly preferred.

Unless otherwise provided, the term "C₁-C₄ aliphatic alcohol" means an alcoholic derivative of general formula R-OH, wherein R represents a linear or branched aliphatic chain, containing from 1 to 4 carbon atoms, such as: methyl, ethyl, iso-propyl and the like.

The reaction can be achieved by mixing the reactive under stirring at room temperature (e.g. at a temperature comprised from 15 to 40° C), for a period of time generally comprised from 1 to 3 hours.

All the reagents are commercially available on the market and known to the person skilled in the art.

As above reported, the present anionic derivatives of formula (I) can be used as medicament, preferably as antiviral, antimycotic or, even more preferably, as antibacterial agent, as demonstrated e.g. in the herein enclosed experimental part. In the enclosed example 6, in particular, some preliminary tests have been carried out by the Applicant against a series of microbic species. The results clearly show the antibacterial activity of the derivatives of the invention, as also confirmed in Fig. 8.

The present derivatives are preferably for the use in the treatment of: cutaneous irritations, inflammations, abrasions, excoriations and/or burns. In a still preferred embodiment, the anionic derivatives (I), preferably anionic silica derivatives, are used in the treatment of diseases selected from: acne, herpes, bed sores, and skin ulcers, preferably decubitus ulcers.

In this respect, in fact, when the derivative are used as medical device, they can be applied to the interested site to form an efficacious film or a barrier, against external microbes, substantially avoiding any infection on the affected skin.

The derivatives are also suitable for the use as a skin cicatrizating agent.

When the anionic derivatives (I) are employed as antibacterial agent, they are preferably used for the treatment of diseases due to at least one of the microbes selected from: HSV-1 (Herpes Simplex Virus-1), Adenovirus, Poliovirus, Pseudomonas aeruginosa, Enterococcus faecalis, Escherica coli, Salmonella enteridis D1, Listeria monocytogenes, Staphylococcus aureus MR, Staphylococcus aureus MS, Streptococcus pyogenes, Streptococcus salivarius, Streptococcus mitis, Candida albicans, Aspergillus niger. Advantageously, the derivatives of the invention may be added themselves to a known pharmaceutical composition, preferably to a topical-treatment composition. By that it is possible to obtain topical-treatment products, preferably medical devices, for example in the form of a cream, a gel or even a powder, endowed with antibacterial, antimycotic, virucidal, and/or sporicidal properties.

Furthermore, the silicon or titanium dioxide derivatives having the herein reported general formula (I), can also be added to known active ingredient, e.g. which have been reported to promote wound healing by increasing collagen content and granulation tissue, such as famotidine or nizadine (for a general reference see e.g. K.S. Rao et al. Inian J. Med. Res. 125, Feb 2007, 149-154).

Owing also to this peculiarity of the present derivatives of formula (I), it is possible to obtain topical-treatment products having antiviral, antibacterial, and antimycotic properties, together with other curative properties, basically depending on the pharmaceutical profile of the drug or composition they are added to. Therefore, according to a further aspect, the anionic derivatives (I), preferably wherein M=Si, are also useful as additives for pharmaceutical compositions, thus allowing for instance the preparation of very efficacious topical-treatment products, having multiple or enhanced pharmaceutical properties.

Moreover, in the preparation of a pharmaceutical composition of the invention, it is not required any particular precautions when compared to the commonly used processes for preparing topical-treatment products. Hence, by providing the adding phase for adding the present derivatives of formula (I), known processes for producing topical-treatment products may be used to prepare topical-treatment products having antibacterial, antimycotic, virucidal and/or sporicidal properties, comprising the present derivatives (I). Therefore, according to a further aspect of the invention, there is provided a pharmaceutical composition comprising the derivative of formula (I), preferably wherein M=Si, in admixture with at least one physiologically acceptable carrier or excipient, and optionally, in admixture with at least another active ingredient.

Conveniently, the physiologically acceptable carriers and/or excipient of the present composition, can be selected among those commonly used in the art, in particular those used for products classified as medical devices, cosmetics or that are included in the food safety regulation. As general examples, when the composition is in the form of a cream, said excipients can be suitably selected from: Vaseline, liquid paraffin, stearyl alcohol, polyethylene glycol stearate and the like. Similarly, if the composition is in the form of a gel, the present derivatives of formula (I) can be admixed with, e.g., glycerine, amidopropylene glycol, magnesium or aluminium silicate and the like.

As formerly indicated, the present composition is preferably for the use as topical-treatment product, even more preferably as medical device, e.g. in the form of cream, powder, spray gel, gel or foam. Still more preferably, the composition is used for treating: acne, herpes simplex, wounds, decubitus ulcers, cutaneous irritations, inflammations, abrasions, excoriations and/or burns.

As above described in details, the present invention provides negatively charged silica or titanium dioxide particles, used for the preparation of derivatives of general formula (I):

(MO₂)⁻ (A^{m+})_{y} (I)

wherein the anionic particles (MO₂)⁻ are self-assembled by electrostatic interaction to two positively charged antibacterial species (A^{m+}), which include chlorhexidine cations, and cationic silver complexes of formula Ag-L, self-assembled by electrostatic forces to the surface of said anionic particles.

The benefit of such antibacterial derivatives is at least two fold: binding electrostatically the active species A^{m+} to the negatively charged (preferably silica) substrate, allows the preparation of antibacterial formulations, for example in the form of powders, creams or gel, where the active specie can also act as a barrier and is therefore very useful for the realization of Medical Devices for topical use, whereby the release of the active ingredient to the tissue should be retained or somehow controlled.

In addition, the use of the anionic substrate allows the simultaneous binding of different antibacterial species (A^{m+}) which may act sinergically, thus increasing their action.

The present invention will now be described by way of examples, which are intended to better illustrate the invention, without limiting its scope.

### EXPERIMENTAL PART

### Example 1 (not part of the present invention): Preparation of (SiO₂)⁻(A^{m+})_{y}, where A^{m+} is the NH₄⁺ cation.

The general procedure reported allows to obtain negatively charged silica particle of different size, depending by the proportion between reaction mixture components such as Water, ethanol, ammonia and tetraethoxysiliane (TEOS) or tetrapropoxysilane or tetrabuthoxysilane or tetrapentoxysilane. Ammonia was used as the catalyst. In many cases, it can be applied by adding saturated alcoholic solutions of ammonia to the reaction vessel. In other cases, it can be added directly in aqueous solution.

Pure ethanol alcohol or alcohol mixtures and saturated alcoholic ammonia solution or aqueous ammonia and water are mixed in Erlenmeyer flasks with ground stoppers. Subsequently the alkyl silicate can be added and the flasks are mounted either on a shaker or in a water bath under ultrasonic vibration. The preparations can also be done maintaining the reaction mixture under magnetic stirring.

After addition for example of TEOS, the appearance of white silica is observed within a few more minutes. The reaction can be continued for 120-240 minutes after that the precipitate can be filtered off, washed several times with ethanol and acetone, until neutrality of the washings, and dried in an oven at 60-100 °C for 24 h.

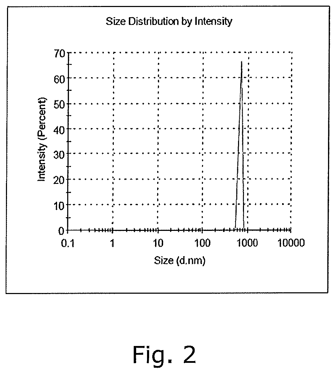

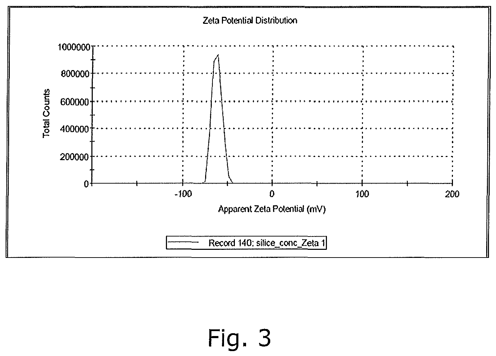

Preferably the following proportion between reactants is used: 95 % Ethanol, 350 ml; distilled water, 250 ml; concentrated ammonium hydroxide, 100 ml; TEOS 300 ml. In these conditions the amount of dry powder obtained is of the order of 82 - 83 gr. Elemental analysis of the powder gives 0.32%N, which considering the atomic weight of nitrogen, allow to infer the presence of ca 0.023 negative charges/100 gr of product which are carried by surface Si-O⁻ groups. In repeated preparations, maintaining the above reported proportion between reactants the % of Nitrogen was observed in the range 0.30-0.40%. Size analysis of the particles composing the powder was done by using a Zetasizer Malvern Instrument 7.01. Measurements were performed on silica powder suspensions in neutral water after ultrasound irradiation for 30 min. The average size distribution obtained was in the range 680 ± 80 nm (Fig. 2) and the Zeta potential was -62 ± 5 V (Fig. 3).

### Example 2: Preparation of (SiO₂)⁻(A^{m+})_{y}, where A^{m+} is the Chlorhexidine cation.

20 gr of anionic silica prepared according to Example 1 were suspended in a 200 ml solution containing 160 ml of ethanol and 40 ml of a 20 % Chlorhexidine Digluconate solution in water. After 30 min of stirring, 100 ml of acetone were added and the suspension kept under stirring for additional 30 min. The solid product was filtered off, dried first under vacuum and then in an oven at 50 °C for 12 h. 25.7 gr of final product were obtained.

Elemental analysis gave a C% of 10% which corresponds to about 20% of chlorhexidine in the product.

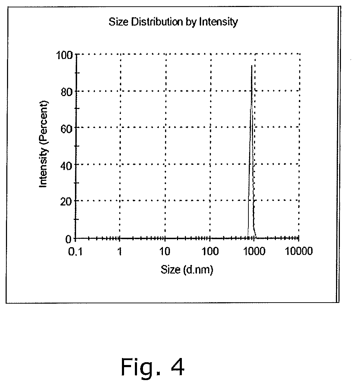

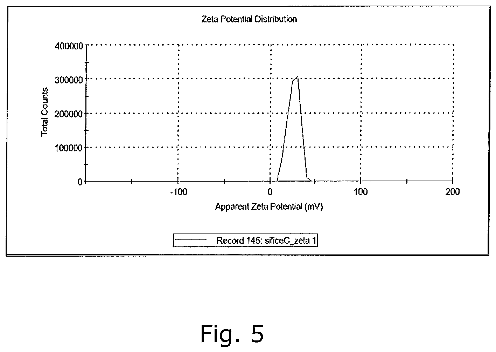

Size analysis of the particles composing the silica-chlorhexidine product gave an average size distribution of 840 ± 90 nm (Fig. 4) and a Zeta potential was + 25 ± 6 V (Fig. 5), consistent with the binding of the chlorhexidine dication to the negatively charged silica surface.

### Example 3: Preparation of (SiO₂)⁻(A^{m+})_{y}, where A^{m+} is the cationic Silver complex Ag-L, with L = 2-mercapto-4-methylpyrimidine Hydrocloride.

20 gr of anionic silica prepared according to Example 1 were suspended in 200 ml of an ethanol solution containing dissolved 81.3 mg of the ligand (2-mercapto-4methylpyrimidine Hydrochloride). The suspension was kept under stirring for ca 2 h after that a 84.9 mg amount of AgNO₃, dissolved in 50 ml of methanol, was added. The color of the white suspension turns to yellow after addition of the silver ions indicating silver complex formation. Stirring was maintained for additional 30 min after that the solid was filtered off and dried in an oven at 50 °C for 4 h.

Silver analysis performed on the solid product by atomic absorption spectroscopy gave 0.27 % Ag and 0.077% S, confirming quantitative binding of the positively charged silver complex to the anionic silica.

### Example 4: Preparation of (SiO₂)⁻(A^{m+})_{y}, where A^{m+} represents both the cationic Silver complex Ag-L, with L = 2-mercapto-4-methylpyrimidine Hydrocloride, as well as the Chlorhexidine cation

The product was prepared with the procedure outlined in Example 2 by using as a starting material the SiO₂-Silver Complex adduct prepared according to Example 3.

Elemental analysis of the product gave 0.25% Ag, 0.073% S, 11% C, consistent with quantitative formation of the Si02-Chlorhexidine-Silver complex.

### Example 5 (not part of the present invention): Preparation of (SiO₂)⁻(A^{m+})_{y}, where A^{m+} is Polyhexamethylene Biguande Hydrochloride (PHMB).

10 gr of anionic silica prepared according to Example 1 were suspended in a 100 ml solution composed by 80 ml of 95% EtOH and 20 ml of 20% PHMB in water. After 30 min of stirring, 200 ml of acetone were added and the stiring kept for additioal 30 min. The solid was filtered off, air dried and then dried in an oven at 50 °C for 12 h giving 12.5 gr of product.

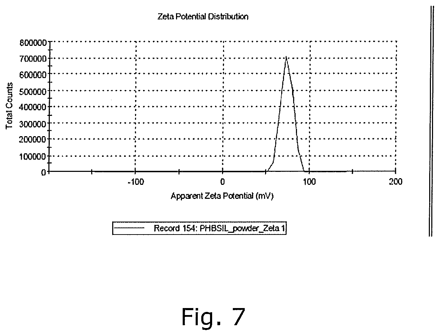

Size analysis of the SiO₂ particles with the electrostatically bound polymeric PHMB gave an average size distribution of 3000 ± 360 nm (Fig. 7) and a Zeta potential was + 73 ± 7 V (Fig. 8), consistent with surface binding of the positively charged polymeric cation.

### Example 6: Antibacterial activity of the powders

The antibacterial activity of the powders was tested against the following microbic species

| | |
|---|---|
| *Pseudomonas aeruginosa* | ATCC 15442 |
| *Staphylococcus aureus* | ATCC 6538 |
| *Escherichia coli* | ATCC 10536 |
| *Enterococcus hirae* | ATCC 10541 |
| *Candida albicans* | ATCC 10231 |

which were purchased from Diagnostic International Distribution SpA.

A mixture of the different microorganism with concentrations in the range 1.5x10⁶ - 5.0x10⁶ for each species was prepared. 100 µl of the mixture was seeded in Petri capsule containing (Tryptone Soya Agar).

Samples of the powders, based on anionic silica with adsorbed the different active species, were immediately put inside the Petri capsule which were then incubated at 37° C for 24 h. After this time the capsule were examined evaluating the colonies proliferation and the inhibition ring surrounding the antibacterial powder.

Fig 8 shows that while no inhibition ring is observed in the case of the anionic silica powder where the counter ion is ammonium (Fig.8A), in the other investigated cases, where antimicrobial cations are adsorbed, the size of the inhibition rings increase progressively in the order of adsorbed cations:
Silver Complex (Fig. 8B) < Chlorhexidine (Fig 8C) < Silver Complex+Chlorhexidine (Fig. 8D), with a more than additive increase in the latter case.

Since these experiments were performed by using comparable amounts of powders and maintaining the silver and chlorhexidine content at comparable level The increased thickness in Fig 8D testify a synergic action between the chlorhexidine and silver cations adsorbed on the same silica particles which enhance their antimicrobic activity of each component.

## Claims

1. A derivative of formula (I):
(MO₂)⁻ (A^{m+})_{y} (I)
wherein:
M is a Si or Ti atom;
(MO₂)⁻ represents an anionic dioxide particle comprising tetrahedral Silicon or octahedral Titanium atoms interconnected through bridging oxygen atoms,
A^{m+} represents a chlorhexidine cation and a cationic silver complex of formula Ag-L, having L preferably selected from: optionally substituted 8-mercapto-quinoline hydrochloride and 2-mercapto-4-(C₁-C₆Alk)pyrimidine hydrochloride, A^{m+} being linked to the anionic dioxide particle by electrostatic interaction, wherein m represents the charge of said active species, and is comprised from 1 to 20, preferably from 1 to 10,
Y represents the number of different active species A^{m+} co-adsorbed to the same anionic silicon or titanium dioxide particle, and it is equal to 2.

2. The derivative of formula (I) according to claim 1, wherein M=Si.

3. The derivative of formula (I) according to claim 1 or 2, **characterized by** the fact that the anionic silicon or titanium dioxide particles have an average diameter of at least 200nm, preferably from 500nm to 1µm.

4. The derivative of formula (I) according to claim 1, wherein L is 2-mercapto-4-methylpyrimidine hydrochloride.

5. The derivative of formula (I) according to claim 1, wherein A^{m+} is chlorhexidine digluconate and Ag-L, wherein L is 2-mercapto-4-methylpyrimidine hydrochloride.

6. The derivative of formula (I) according to any one of the preceding claims, for use as a medicament.

7. The derivative of formula (I) for use, according to claim 6, as antibacterial, antimycotic, antiviral or sporicidal agent.

8. The derivative of formula (I) for use according to claim 6 or 7, in the treatment of cutaneous irritations, inflammations, abrasions, excoriations and/or burns.

9. The derivative of formula (I) for use according to any one of claim 6 to 8, in the treatment of: acne, herpes, bed sores, skin ulcers, preferably decubitus ulcers.

10. The derivative of formula (I) for use according to any one of claim 6 to 9, as a skin cicatrizating agent.

11. The derivative of formula (I), for use according to any one of claim 6 to 10 as antibacterial agent for treatment of diseases due to at least one of the microbes selected form: HSV-1 (Herpes Simplex Virus-1), Adenovirus, Poliovirus, Pseudomonas aeruginosa, Enterococcus faecalis, Escherica coli, Salmonella eneridis D1, Listeria monocytogenes, Staphylococcus aureus MR, Staphylococcus aureus MS, Streptococcus pyogenes, Streptococcus salivarius, Streptococcus mitis, Candida albicans, Aspergillus niger.

12. A pharmaceutical composition comprising the derivative of formula (I) as described in any one of claim 1 to 5, in admixture with at least one physiologically acceptable excipient and/or carrier, and, optionally, with at least another pharmaceutical active ingredient.

13. The pharmaceutical composition according to claim 12, in the form of a cream, a gel, a foam or a powder.

14. The pharmaceutical composition according to claims 12-13 for topic administration.

15. A process for the preparation of the derivative of formula (I), as defined in any one of claim 1 to 5, comprising the electrostatic interaction of anionic silica particles of general formula (MO₂)⁻, wherein M is a Si or Ti atom, having a ps of at least 200nm, with two positively charged A^{m+} species, a chlorhexidine cation and a cationic silver complex of formula Ag-L, in the presence of an alcoholic solvent system.

## Patentansprüche

1. Derivat der Formel (I):
(MO₂)- Am⁺)y (I)
wobei:
M ein Si- oder Ti-Atom ist;
(MO₂) ein anionisches Dioxidteilchen darstellt, umfassend tetraedrische Silicium- oder oktaedrische Titanatome, die über verbrückende Sauerstoffatome miteinander verbunden sind,
A^{m+} ein Chlorhexidinkation und einen kationischen Silberkomplex der Formel Ag-L darstellt, wobei L vorzugsweise ausgewählt ist aus: wahlweise substituiertem 8-Mercapto-chinolin-hydrochlorid und 2-Mercapto-4-(C₁-C₆Alk)pyrimidinhydrochlorid, wobei A^{m+} mit dem anionischen Dioxidteilchen durch elektrostatische Wechselwirkung verbunden ist, wobei m die Ladung der aktiven Spezies darstellt und 1 bis 20, vorzugsweise 1 bis 10 beträgt, Y die Anzahl der verschiedenen aktiven Spezies A^{m+} darstellt, coadsorbiert an das gleiche anionische Silizium- oder Titandioxidteilchen und gleich 2 ist.

2. Derivat der Formel (I) nach Anspruch 1, wobei M=Si.

3. Derivat der Formel (I) nach Anspruch 1 oder 2, **dadurch gekennzeichnet, dass** die anionischen Silicium-oder Titandioxidteilchen einen durchschnittlichen Durchmesser von mindestens 200nm, vorzugsweise von 500nm bis 1µm aufweisen.

4. Derivat der Formel (I) nach Anspruch 1, wobei L 2-Mercapto-4-methylpyrimidinhydrochlorid ist.

5. Derivat der Formel (I) nach Anspruch 1, wobei A^{m+} Chlorhexidindigluconat und Ag-L ist, wobei L 2-Mercapto-4-methylpyrimidinhydrochlorid ist.

6. Derivat der Formel (I) nach einem der vorhergehenden Ansprüche, zur Verwendung als ein Medikament.

7. Derivat der Formel (I) zur Verwendung nach Anspruch 6 als antibakterielles, antimykotisches, antivirales oder sporizides Mittel.

8. Derivat der Formel (I) zur Verwendung nach Anspruch 6 oder 7, bei der Behandlung von Hautreizungen, Entzündungen, Abschürfungen, Exkoriationen bzw. Verbrennungen.

9. Derivat der Formel (I) zur Verwendung nach einem der Ansprüche 6 bis 8 zur Behandlung von: Akne, Herpes, Wundliegen, Hautgeschwüren, vorzugsweise Dekubitusgeschwüren.

10. Derivat der Formel (I) zur Verwendung nach einem der Ansprüche 6 bis 9, als hautvernarbender Wirkstoff.

11. Derivat der Formel (I), zur Verwendung nach einem der Ansprüche 6 bis 10, als antibakterieller Wirkstoff bei der Behandlung von Krankheiten aufgrund von mindestens einer der Mikroben, ausgewählt aus: HSV-1 (Herpes Simplex Virus-1), Adenovirus, Poliovirus, Pseudomonas aeruginosa, Enterococcus faecalis, Escherica coli, Salmonella eneridis D1, Listeria monocytogenes, Staphylococcus aureus MR, Staphylococcus aureus MS, Streptococcus pyogenes, Streptococcus salivarius, Streptococcus mitis, Candida albicans, Aspergillus niger.

12. Pharmazeutische Zusammensetzung, umfassend das Derivat der Formel (I) wie in einem der Ansprüche 1 bis 5 beschrieben, in der Mischung mit mindestens einem physiologisch akzeptablen Hilfsstoff und/oder Träger und wahlweise mit mindestens einem anderen pharmazeutischen aktiven Bestandteil.

13. Pharmazeutische Zusammensetzung nach Anspruch 12 in Form von einer Creme, einem Gel, einem Schaum oder einem Pulver.

14. Pharmazeutische Zusammensetzung nach den Ansprüchen 12-13 zur topischen Verabreichung.

15. Verfahren zur Herstellung des Derivats der Formel (I), wie definiert in einem der Ansprüche 1 bis 5, umfassend die elektrostatische Wechselwirkung von anionischen Siliciumdioxidteilchen der allgemeinen Formel (MO₂)-, wobei M ein Si- oder Ti-Atom aufweisend ein ps von mindestens 200nm mit zwei positiv geladenen A^{m+}-Spezies, einen Chlorhexidinkation und einen kationischen Silberkomplex der Formel Ag-L in Gegenwart eines alkoholischen Lösungsmittelsystems ist.

## Revendications

1. Dérivé de formule (I) :
(MO₂)⁻ A^{m+})_{y} (I)
où:
M est un atome de Si ou de Ti ;
(MO₂) représente une particule de dioxyde anionique comprenant des atomes de silicium tétraédriques ou de titane octaédriques interconnectés par des atomes de pontage d'oxygène,
A^{m+} représente un cation de chlorhexidine et un complexe cationique d'argent de formule Ag-L, ayant L de préférence choisi à partir de chlorhydrate 8-mercapto-quinoléine et chlorhydrate de pyrimidine 2-mercapto-4-(C₁-C₆Alk) facultativement substitué, A^{m+}étant lié à la particule de dioxyde anionique par interaction électrostatique, dans lequel m représente la charge desdites espèces actives et est compris entre 1 et 20, de préférence entre 1 et 10, Y représente le nombre de différentes espèces actives A^{m+} co-absorbées à la même particule de silicium anionique ou de dioxyde de titane, et est égal à 2.

2. Dérivé de formule (I) selon la revendication 1, dans lequel M = Si.

3. Dérivé de formule (I) selon la revendication 1 ou 2, **caractérisé en ce que** les particules de silicium anioniques ou de dioxyde de titane ont un diamètre moyen d'au moins 200 nm, de préférence de 500 nm à 1 µm.

4. Dérivé de formule (I) selon la revendication 1, dans lequel L est un hydrochlorate 2-mercapto-4-méthylpyrimidine.

5. Dérivé de formule (I) selon la revendication 1, dans lequel A^{m+} est du digluconate de chlorhexidine et de l'Ag-L, dans lequel L est un hydrochlorate 2-mercapto-4-méthylpyrimidine.

6. Dérivé de formule (I) selon l'une quelconque des revendications précédentes, à utiliser comme médicament.

7. Dérivé de formule (I) à utiliser selon la revendication 6, comme antibactérien, antimycotique, antiviral ou agent sporicide.

8. Dérivé de formule (I) à utiliser selon la revendication 6 ou 7, dans le traitement des irritations cutanées, des inflammations, des éraflures, des excoriations et/ou des brûlures.

9. Dérivé de formule (I) à utiliser selon l'une quelconque des revendications de 6 à 8, dans le traitement de : l'acné, de l'herpès, des escarres de décubitus, des ulcères cutanés, de préférence des ulcères de décubitus.

10. Dérivé de formule (I) à utiliser, selon l'une quelconque des revendications de 6 à 9, comme agent cicatrisant de la peau.

11. Dérivé de formule (I) à utiliser, selon l'une quelconque des revendications de 6 à 10, comme agent antibactérien pour le traitement de maladies provoquées par au moins l'une des bactéries sélectionnées parmi celles qui suivent : HSV-1 (virus herpès simplex de type 1), Adénovirus, Poliovirus, Pseudomonas aeruginosa, Enterococcus faecalis, Escherichia coli, Salmonella enteritidis D1, Listeria monocytogenes, Staphylocoque doré MR, Staphylocoque doré MS, Streptocoque pyogène, Streptococcus salivarius, Streptococcus mitis, Candida albicans, aspergillus niger.

12. Composition pharmaceutique, comprenant le dérivé de formule (I) tel que décrit dans l'une quelconque des revendications de 1 à 5, mélangée avec au moins un excipient et/ou un vecteur de médicament physiologiquement acceptable, et, éventuellement avec au moins un ingrédient pharmaceutique actif supplémentaire.

13. Composition pharmaceutique selon la revendication 12, sous forme de crème, de gel, de mousse ou de poudre.

14. Composition pharmaceutique selon les revendications 12-13, destinée à une administration topique.

15. Procédé de préparation du dérivé de formule (I), tel que défini dans l'une quelconque des revendications de 1 à 5, comprenant l'interaction électrostatique de particules de silicium anioniques de formule générale (MO₂)-, dans lequel M est un atome de Si ou de Ti ayant une ps d'au moins 200 nm, avec deux espèces à charge positive A^{m+}, un cation de chlorhexidine et un complexe cationique d'argent de formule Ag-L, en présence d'un système solvant alcoolique.
